# EUROPEAN PATENT APPLICATION

(11) **EP 3 909 604 A1**
(43) Date of publication of application: **17.11.2021**
(21) Application number: 20174523.9
(22) Date of filing: 13.05.2020
(51) Int. Cl.: A61K 39/12

(54) **COMPOSITION FOR THE TREATMENT AND/OR PREVENTION OF MARINE MOLLUSC VIRAL INFECTION**

(71) Applicant: IFREMER, 29280 Plouzane (FR)
(72) Inventor: The designation of the inventor has not yet been filed
(74) Representative: Barbot, Willy

(57) **Abstract**

The present invention concerns a composition comprising at least one non-infectious virus particle for preventing and/or treating a viral infection in a marine mollusc.

## Description

### Domain of the invention

The present invention relates to the prevention and/or treatment of marine mollusc's viral infection.

### Prior art

Marine mollusc production is increasingly important to global food supply. Today, global bivalve production represents nearly 15 million tons.

Now, these productions have shown a slow growth essentially because of infectious diseases. As an example, irido-like virus infections led to the almost total extermination of the oyster *C. angula* in European Atlantic waters in the early 1970's. Since the early 1990's, viruses belonging to the Herpesviridae family have been associated with high mortality rate of *C. gigas* larvae and spat in Europe, but also in New Zealand, Australia, China, USA and Mexico.

These examples highlight the adverse effect of infectious diseases on marine mollusc production.

The acknowledgement of these infectious diseases as a major driver for marine mollusc production thus fueled interest in the immunity of these animals.

In fact, the understanding of immune mechanisms and response to infections represents a great advantage for the obtaining of disease-resistant cultured bivalves.

Previous studies showed that bivalves possess distinct and specific antiviral and antibacterial responses (GREEN et al., Veterinary research, 47:72, 2016).

In case of a viral infection, the involved pathway oyster is notably under the control of dsRNA. Accordingly, poly (I:C) as been identified as a promising antiviral response elicitor.

In contrast, the bivalve subjected to a bacterial infection displayed a distinct response involving genes associated with a broad range of functions including cellular proliferation, adhesion, migration and immune response (e.g. big defensin).

As evidence of such differences:
- oysters primarily stimulated by killed bacteria exhibited stronger immune responses when they are infected by *Vibrio splendidus* (LI et al., Dev. Comp. Immunol., vol.71, p:59-69, 2017), whereas such primary stimulation with killed bacteria was not efficient in protecting oyster against virus infection (GREEN & MONTAGNANI, Fish Shellfish Immunol., vol.35, p:382-388, 2013).
- poly (I:C)induce an antiviral response in oyster, whereas it fails to protect a pathogenic bacteria (LAFONT et al., Sci. Rep., vol. 7, p: 13143, 2017).

### Summary of the invention

The inventors have made a surprising discovery during experiments related to the characterization of poly (I:C) oyster associated immune response.

Whereas dsDNA (LAFONT et al., abovementioned, 2017) or killed bacteria (i.e. ds bacterial DNA and proteins; GREEN & MONTAGANI , abovementioned, 2013) did not induced any antiviral response, the use of inactivated virus as a negative priming control has resulted surprisingly in the obtaining of an antiviral response, which antiviral response was similar to the poly (I:C) one.

Whereas the mechanism underlying such immunity is not still clear, this discovery enables to envisage the vaccination of marine mollusc against virus infection using compositions comprising inactivated virus.

Accordingly, the present invention concerns a composition comprising at least one non-infectious virus particle for preventing and/or treating a viral infection in a marine mollusc, preferably the virus particle is specific to the viral infection.

In a third object, the present invention concerns a method for preventing and/or treating a viral infection in a marine mollusc comprising the step of administrating to said marine mollusc a composition comprising an effective amount of at least one non-infectious virus particle.

Preferably, the virus particle is specific to the viral infection.

### Description of the figures

The figure 1 summarizes the experiment protocol.
The figure 2 shows the survival rates of the different oyster sub-groups with time.
The figure 3 shows the virus replication in the different oyster sub-groups with time.

### Detailed description of the invention

As used "herein", the term marine mollusc refers to bivalve or marine gastropods.

As used herein, the term "marine gastropods" may denote an abalone, a conch, a periwinkle, or a whelk.

As used herein, the term "bivalve" may denote a clam, an oyster, a cockle, a mussel, or a scallop.

Preferably, said marine mollusc is a bivalve and, most preferably, said marine mollusc is an oyster.

The oysters are members of the family Ostreidae. This family includes the edible oysters, which mainly belong to the genera *Ostrea, Crassostrea, Ostreola, Magallana, Saccostrea,* and *Pteriidae* (e.g. *Pinctada*).

Now and in a still preferred embodiment, said oyster belong to the genera *Crassostrea,* and is preferably *Crassostrea gigas.*

In relation with the term "virus", it refers to a virus that may infect a marine mollusc or accumulate in a marine mollusc. As an example of such viruses, one can cites viruses belonging to a wide range of viral families including *Herpesviridae, Papoviridae, Togaviridae, Retroviridae, Reoviridae, Norovirus, Birnaviridae* and *Picornaviridae* (For a review, see. ARZUL et al., Journal of Invertebrate Pathology, vol.147, p:118-135, 2017).

Among these virus families, most of information concerns the *Herpesviridae* family. In fact, herpesviruses are associated to mortality outbreaks resulting in high losses in several marine mollusc species worldwide, including the Pacific oyster *C gigas.*

Thus, in a preferred embodiment, said virus is a herpesvirus.

A herpesvirus has been purified from naturally infected larval Pacific oysters collected in 1995 in a French commercial hatchery (LE DEUFF & RENAULT, J. Gen. Virol., vol.80, p: 1317-1322, 1999). This virus has been classified as ostreid herpesvirus type-1 (Genome Accession number AY509253). As herpesvirus, the OsHV-1 is a double-strand DNA of about 207 kb.

Accordingly, said virus is preferably ostreid herpesvirus type-1 (OsHV-1).

As used herein, a virus particle means a virus particle containing a viral genome. Now, a virus particle also means a virus particle containing a mutated viral genome (*e.g.,* by nucleic acid mutation, substitution or insertion) or a virus particle not containing any viral genome and viral proteins resulting in the production of non-infectious virus particles.

Advantageously, said virus particle is an inactivated virus particle. If not necessary for non-replicative particles, such inactivation is necessary for replicative virus particles.

As used herein "an inactivated virus particle" means a viral particle, which is no more infectious.

Surprisingly, the inventors have established that virus UV inactivated virus induces specific protection.

Methods for inactivation of viral particles or recombinant virus particles are well known from one of skill in the art. Non-limiting examples of viral inactivation include chemical inactivation such as formalin, taurine chloramine, formaldehyde, paraformaldehyde, propiolactene, beta-propiolactone (REMUNE) or alditriol-2) treatment, thermal inactivation, physical inactivation such as U.V or gamma irradiation or microwave exposure, and combinations thereof.

According to a specific embodiment, the composition of the invention may also comprise an adjuvant.

As used herein, the term "adjuvant" refers to a substance used in combination with virus particle that produced more immunity than the virus particle alone.

In another preferred embodiment, the composition of the invention further comprises at least one absorption-promoting agent.

Absorption-promoting agents are well known from one of skill in the art. As examples, one can cites surfactants such as polyoxyethylene derivatives of fatty acid partial esters of sorbitol anhydrides (e.g., Tween® 80, Polyoxyl 40 Stearate, Polyoxyethylene 50 Stearate, polyoxyethylene-9-lauryl ether and Octoxynol), mixed micelles, enamines, sodium salicylate, glycerol esters of acetoacetic acid (eg., glyceryl-1,3-diacetoacetate or 1,2-isopropylideneglycerine-3-acetoacetate), clyclodextrin or beta-cyclodextrin derivatives (eg., 2-hydroxypropyl-beta-cyclodextrin and heptakis(2,6-di-O-methyl-beta-cyclodextrin)), medium-chain fatty acid such as mono- and diglycerides (eg., sodium caprate-extracts of coconut oil, Capmul), or triglycerides (eg., amylodextrin, Estaram 299, Miglyol 810), polymers such as carboxymethylcellulose, carbopol, polycarbophil, tragacanth or sodium alginate.

The composition may comprise one or more additives (e.g., diluents, excipients, stabilizers, preservatives). See, generally, Ullmann's Encyclopedia of Industrial Chemistry, 6th Ed. (various editors, 1989-1998, Marcel Dekker); and Pharmaceutical Dosage Forms and Drug Delivery Systems (ANSEL et al., 1994, WILLIAMS & WILKINS).

The composition of the invention may be administrated once or several time.

Advantageously, the interval between said consecutive administrations is comprised between 15 days and 3 months, preferably between 1 and 2 months.

According to the present invention, an "effective amount" of a composition is one which is sufficient to achieve a desired biological effect, in this case at least one of antiviral response to virus infection. The ranges of effective doses provided below are not intended to limit the invention and represent preferred dose ranges.

Generally speaking, the dosage for an oyster will be from about 10³ - 10⁸ virus particles per dose with 10⁶ - 5x10⁷ preferred.

The invention is now illustrated by the following non-limiting examples.

Experiments have been performed on specific pathogen free (SPF) (i.e. free of mortality, negative for OsHV-1 detection and very low Vibrio sp. bacteria concentrations74) juvenile 10 months old *C. gigas* oysters.

OsHV-1 inoculums (or virus homogenates) were prepared according to SCHIKORSKI et al. (Virus Res., vol.155, p:28-34, 2011) from moribund oysters experimentally infected with OsHV-1 in previous trials and frozen at -80 °C. Viral DNA loads (OsHV-1 µvar genomes copies.µL-1) in inoculums were estimated by qPCR. Viral inoculums were confirmed to be free of cultivable bacteria by plating 40 µl on LB NaCl agar plates. Control inoculums (control homogenate) were prepared following the same protocol from healthy naive oysters showing no detectable amount of viral DNA.

Oysters were injected either with 100µL of viral inoculum UV-treated or not containing 1,37.10⁷cp/µL.

For each priming condition, animals were previously anesthetized in hexahydrate MgCl₂. Oysters were injected using 26-gauge needle attached to a multi-dispensing hand pipette with a Poly(I:C) solution (PIC) in the adductor muscle to spread into the circulatory system. Injection of UV inactivated OsHV-1 inoculum as ds DNA (virus UV), UV inactivated control inoculum (no virus UV), and sterile filtered seawater (FSW) were used as a further control.

24 h after priming, each oyster group was exposed to seawater contaminated with OsHV-1 (/virus) or to non-contaminated seawater (/FSW).

Each condition was then separated in two subgroups with one for mortality monitoring and one for sampling.

Sampling consisted in individually removing shells of each oyster with a sterile scalpel blade and snap-froze the whole oyster in liquid nitrogen by pool of three animals. The oysters were stored at -80 °C before grinding to powder and nucleic acid purification. Then, viral genomic DNA per ng of oyster was determined as in BATISTA et al. (mentioned. Virol., vol(139), p:1-11, 2007).

Mortality was followed daily and dead spats were removed from tanks and stored at -80 °C. Survival rate data were analyzed for statistical differences between treatments by log rank test on Kaplan-Meier survival curves.

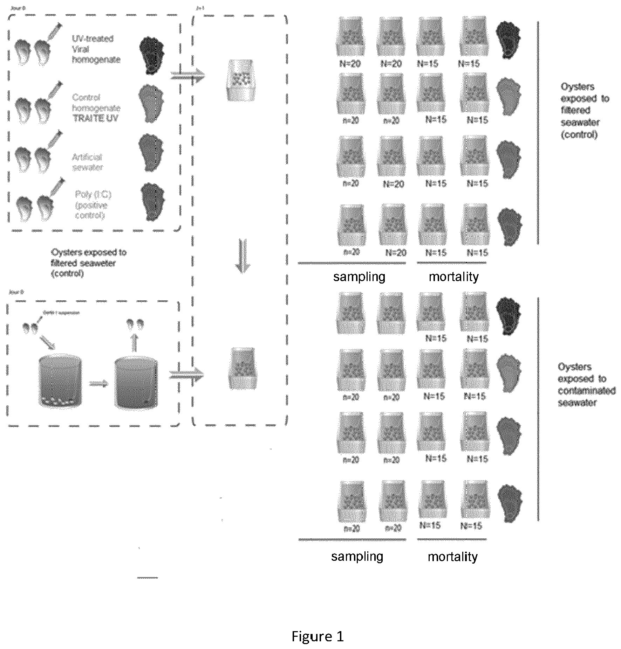

The protocol is summarized in Figure 1 with the number of oysters in each subgroup.

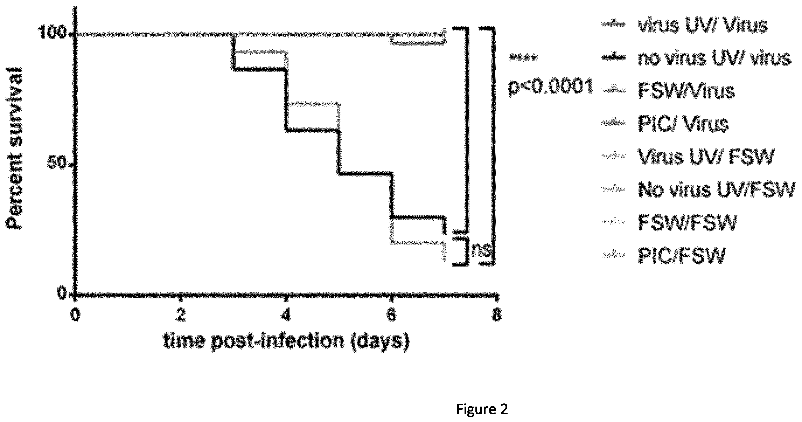

The figure 2 shows the oyster survival (%)after a primary exposure to UV treated OsHV-1(virus-UV in black), poly (I:C) (PIC, in grey), UV-treated non-viral suspension (no virus UV, in dashed line) or FSW (dotted line) and a secondary exposure to OsHV-1 or FSW. Controls reaching 100% survival (poly(I:C) and oysters challenged with FSW appear hidden and merged behind the non-treated control line. Mortalities in each group of 30 oysters (15 per tank) were monitored for 10 days after infection. **** indicates pvalue <0.0001; log-rank test (n=30).

The table 1 summarized the survival rates obtained in two separate experiments.

**Table 1**

| Condition | Oyster number | 1^{st} experiment | 2^{nd} experiment |
|---|---|---|---|
| Poly (I:C)/virus | 30 | 100 | 100 |
| virus UV/virus | 30 | 96.7 | 100 |
| No virus UV/virus | 30 | 23.3 | 56.7 |
| FSW/virus | 30 | 13.3 | 46.7 |
| Poly (I:C)/FSW | 30 | 100 | Not tested |
| virus UV/FSW | 30 | | Not tested |
| No virus UV/FSW | 30 | | Not tested |
| FSW/FSW | 30 | | Not tested |

The results established that, while the population of the negative groups (FSW and no virus UV) decreased following its exposition to contaminated seawater (/virus), the oysters population primed Poly (I:C) (PIC) was stable 7 days post-infection.

Now, and unexpectedly, the results also established that the oyster population primed with inactivated OsHV-1 virus (virus UV), was also stable 7 days post-infection.

Accordingly, UV inactivated virus seems to be an antiviral response elicitor as poly (I:C).

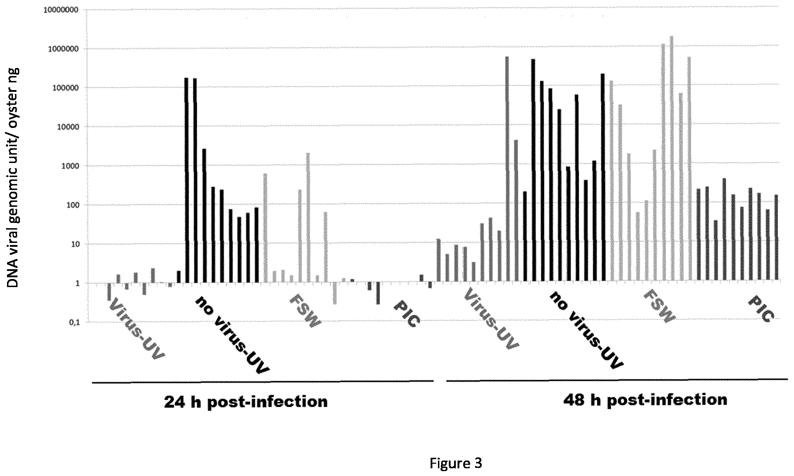

The figure 3 shows OsHV-1 DNA loads 24h (T2) and 48h (T3) after the secondary exposure to OsHV-1 (CSW for contaminated seawater). DNA loads were estimated by real time PCR in 10 individual oysters for each condition. Mann-Whitney T test, ** pvalue < 0.05.

Again, the results established that, while the virus replication increase in the negative groups (FSW and no virus UV), said virus replication remains low and stable in the oysters population primed Poly (I:C) (PIC) and with inactivated virus.

Thus, the inventors initiate a protocol of "pseudo-vaccination" of oyster larvae using inactivated viral suspension or inactivated contaminated seawater

For these experiments, 3ml of viral suspensions are concentrated at 10⁵ to 10⁸ DNA copies viral/mL.

These viral suspensions and 10ml of contaminated seawater (CSW) bearing 10⁸ DNA copies viral/mL are subjected to UV illumination for inactivating virus.

Oyster 6 days post-fecundation larvae (i.e. 100 µm) are exposed to inactivated viral suspension during 24 hours or are immersed in inactivated contaminated seawater.

Then, said larvae at a concentration of 50 larvae /mL at 20°C are exposed to a 1/1000 dilution of viral suspension concentrated at 10⁸ viral DNA copies/mL. Then, larvae mortality is monitored during 7 days.

## Claims

1. A composition comprising at least one non-infectious virus particle for preventing and/or treating a viral infection in a marine mollusc.

2. The composition of claim 1, wherein said marine mollusc is a bivalve or a marine gastropod.

3. The composition of claim 2, wherein said marine mollusc is a bivalve selected in the group comprising clams, oysters, cockles, mussels, and scallops.

4. The composition of claim 3, wherein said bivalve is an oyster.

5. The composition of any one of claims 1 to 4, wherein said virus may infect a marine mollusc.

6. The composition of claim 5, wherein said virus belongs to viral families among *Herpesviridae, Papoviridae, Togaviridae, Retroviridae, Reoviridae, Birnaviridae, Norovirus* and *Picornaviridae.*

7. The composition of claim 6, wherein said virus is a herpesvirus.

8. The composition of claim 7, wherein said herpesvirus is ostreid herpesvirus type-1 (OsHV-1).

9. The composition of any one of claims 1 to 8, wherein said virus particle is an inactivated virus particle.

10. The composition of any one of claims 1 to 9, wherein said composition further comprise an adjuvant.
